# EUROPEAN PATENT SPECIFICATION

(11) **EP 1 507 865 B1**
(45) Date of publication and mention of the grant of the patent: **16.07.2008**
(21) Application number: 03732512.3
(22) Date of filing: 30.05.2003
(51) Int. Cl.: C12N 15/90, C12N 9/22, C12N 15/85, A61K 38/00, C12N 5/06, A01K 67/027

(54) **THE FROG PRINCE, A TRANSPOSON VECTOR FOR GENE TRANSFER IN VERTEBRATES**
"FROG-PRINCE" - EIN TRANSPOSONVEKTOR FÜR DEN GENTRANSFER BEI WIRBELTIEREN
FROG PRINCE, UN VECTEUR DE TRANSPOSONS DESTINE A TRANSFERER DES GENES DANS DES VERTEBRES

(30) Priority: 29.05.2002 DE 10224242
(43) Date of publication of application: 23.02.2005
(73) Proprietor: MAX-DELBRÜCK-CENTRUM FÜR MOLEKULARE MEDIZIN, 13125 Berlin (DE); Netherland Cancer Institute, 1066 CX Amsterdam (NL)
(72) Inventor: MISKEY, Csaba, 13125 Berlin (DE); IZSVAK, Zsuzsanna, 13125 Berlin (DE); IVICS, Zoltan, 13125 Berlin (DE); PLASTERK, Ronald, NL-3584 CT Utrecht (NL)
(74) Representative: Graf von Stosch, Andreas
(86) International application number: PCT/EP2003/005707
(87) International publication number: WO 2003/100070

(56) References cited:
- WO-A-99/25817
- IVICS Z ET AL: "MOLECULAR RECONSTRUCTION OF SLEEPING BEATUY A TC1-LIKE TRANSPOSON FROM FISH, AND ITS TRANSPOSITION IN HUMAN CELLS" CELL, CELL PRESS, CAMBRIDGE, NA, US, vol. 91, November 1997 (1997-11), pages 501-510, XP000700195 ISSN: 0092-8674
- LAM WAN L ET AL: "Discovery of amphibian Tc1-like transposon families" JOURNAL OF MOLECULAR BIOLOGY, vol. 257, no. 2, 1996, pages 359-366, XP002258637 ISSN: 0022-2836
- PONTECORVO GIOVANNI ET AL: "A novel repeated sequence DNA originated from a Tc1-like transposon in water green frog Rana esculenta" GENE (AMSTERDAM), vol. 261, no. 2, 31 December 2000 (2000-12-31), pages 205-210, XP002258638 ISSN: 0378-1119

## Description

The present invention relates to a transposon-based DNA integration system comprising (a) a transposon which is devoid of a polynucleotide encoding a functional transposase and which comprises a polynucleotide of interest, wherein the transposon comprises inverted repeats having a degree of identity with the repeats within SEQ ID NO: 2 and its inverted repeat, respectively, of at least 90%; and (b) a transposase having at its N-terminus a DNA binding domain comprising the sequences of SEQ ID NO: 3 and 4; or (c) a polynucleotide encoding the transposase of (b). The present invention further relates to the use of transposon-based DNA integration system for the preparation of a medicament for the transfer of a polynucleotide of interest into cells of a vertebrate by introducing the transposon-based DNA integration system of the invention into said cells. In addition, the invention relates to an in-vitro method of effecting RNAi comprising (a) stably introducing a transposon comprising an expression cassette expressing a short interfering RNA and a selectable marker gene as part of the transposon-based DNA integration system of the invention into a cell; (b) selecting for cells expressing the selectable marker, and (c) assessing whether the transcription/translation of the desired gene is effected by RNAi. A further embodiment of the invention is an in-vitro method of gene trapping genes comprising the steps of (a) introducing the transposon-based DNA integration system of the invention into a cell; and (b) assessing for the expression of a selectable marker wherein expression of a selectable marker is indicative of integration of the transposon into a transcibed gene of the cell.

Considerable effort has been devoted to the development of in vivo gene delivery strategies for the treatment of inherited and aquired disorders in humans (somatic gene transfer) as well as for transgenesis of certain vertebrate species for agricultural and medical biotechnology (germline gene transfer). For effective gene therapy it is necessary to: 1) achieve delivery of therapeutic genes at high efficiency specifically to relevant cells, 2) express the gene for a prolonged period of time, 3) ensure that the introduction of the therapeutic gene is not deleterious.

There are several methods and vectors in use for gene delivery for the purpose of human gene therapy (Verma and Somia,1997). These methods can be broadly classified as viral and nonviral technologies, and all have advantages and limitations; none of them providing a perfect solution. In general, vectors that are able to integrate the transgene have the capacity to provide prolonged expression as well. On the other side, random integration into chromosomes is a concern, because of the potential disruption of endogenous gene function at and near the insertion site.

Adapting viruses for gene transfer is a popular approach, but genetic design of the vector is restricted due to the constraints of the virus in terms of size, structure and regulation of expression. Retroviral vectors (Miller, 1997) are efficient at integrating foreign DNA into the chromosomes of transduced cells, and have enormous potential for life-long gene expression. However, the amount of time and financial resources required for their preparation may not be amenable to industrial-scale manufacture. Furthermore, there are several other considerations including safety, random chromosomal integration and the requirement of cell replication for integration. Lentiviral systems, based on the human immunodeficiency virus (HIV) belong to retroviruses, but they can infect both dividing and non-dividing cells. Adenovirus vectors have been shown to be capable of in vivo gene delivery of transgenes to a wide variety of both dividing and non-dividing cells, as well as mediating high level, but short term transgene expression. Adenoviruses lack the ability to integrate the transferred gene into chromosomal DNA, and their presence in cells is short-lived. Thus, recombinant adenovirus vectors have to be administered repeatedly, generating an undesirable immune response in humans, due to the immunogenity of the vector. Adeno Associated Virus (AAV) vectors have several potential advantages to be explored, including the potential of targeted integration of the transgene. One of the obvious limitations of the AAV vehicle is the low maximal insert size (3.5-4.0 kb).

Currently, combination (hybrid) vectors (retroviral/adenoviral, retroviral/AAV, etc.) have been developed that are able to address certain problems of the individual viral vector systems.

Nonviral methods, including DNA condensing agents, liposomes, microinjection and "gene guns" might be easier and safer to use than viruses. However, the efficiency of naked DNA entry and uptake is low, that can be increased by using liposomes. In general, the currently used non-viral systems are not equipped to promote integration into chromosomes. As a result, stable gene transfer frequencies using nonviral systems have been very low. Moreover, most nonviral methods often result in concatamerization as well as random breaks in input DNA, which might lead to gene silencing.

Transposable elements, or transposons in short, are mobile segments of DNA that can move from one locus to another within genomes (Plasterk et al., 1999). These elements move via a conservative, "cut-and-paste" mechanism: the transposase catalyzes the excision of the transposon from its original location and promotes its reintegration elsewhere in the genome. Transposase-deficient elements can be mobilized if the transposase is provided *in trans* by another transposase gene. Thus, transposons can be harnessed as vehicles for bringing new phenotypes into genomes by transgenesis. They are not infectious and due to the necessity of adaptation to their host, they thought to be less harmful to the host than viruses.

DNA transposons are routinely used for insertional mutagenesis, gene mapping, and gene transfer in well-established, non-vertebrate model systems such as *Drosophila melanogaster* or *Caenorhabditis elegans,* and in plants. However, transposable elements have not been used for the investigation of vertebrate genomes for two reasons. First, until now, there have not been any well-defined, DNA-based mobile elements in these species. Second, in animals, a major obstacle to the transfer of an active transposon system from one species to another has been that of species-specificity of transposition due to the requirement for factors produced by the natural host.

*Sleeping Beauty* (SB) is an active Tc1-like transposon that was reconstructed from bits and pieces of inactive elements found in the genomes of teleost fish (Ivics et al., 1997). SB mediates efficient and precise cut-and-paste transposition in fish, frog, and many mammalian species including mouse and human cells (Ivics et al., 1997; Luo et al., 1998; Izsvak et al., 2000; Yant et al., 2000).

However, the efficiency of SB transposition in cell lines derived from different species is variable. For example, the efficiency of SB transposition is rather poor in zebrafish cells (Izsvak et al., 2000), which potentially limits the use of SB in this important vertebrate model organism. This phenomenon is not completely understood, and might be a consequence of several factors. Since SB is a fish element, one possible explanation can be interference by endogenous elements in the zebrafish genome. Therefore, having a palette of different, vertebrate-derived transposons might solve such a problem. The technical problem underlying the present invention thus was to provide alternative transposon-based gene delivery systems to widen the potential of transposons as genomic tools in vertebrates.
Accordingly, the present invention relates to a transposon-based DNA integration system comprising
(a) a transposon which is devoid of a polynucleotide encoding a functional transposase and which comprises a polynucleotide of interest, wherein the transposon comprises inverted repeats having a degree of identity with the repeats within SEQ ID NO: 2 or its inverted repeats, respectively, of at least 90%; and
(b) a transposase having at its N-terminus a DNA binding domain comprising the sequences of SEQ ID NO: 3 and 4 or having a degree of identity of at least 90 % to SEQ ID NO: 3 and/or 4; or
(c) a polynucleotide encoding the transposase of (b).

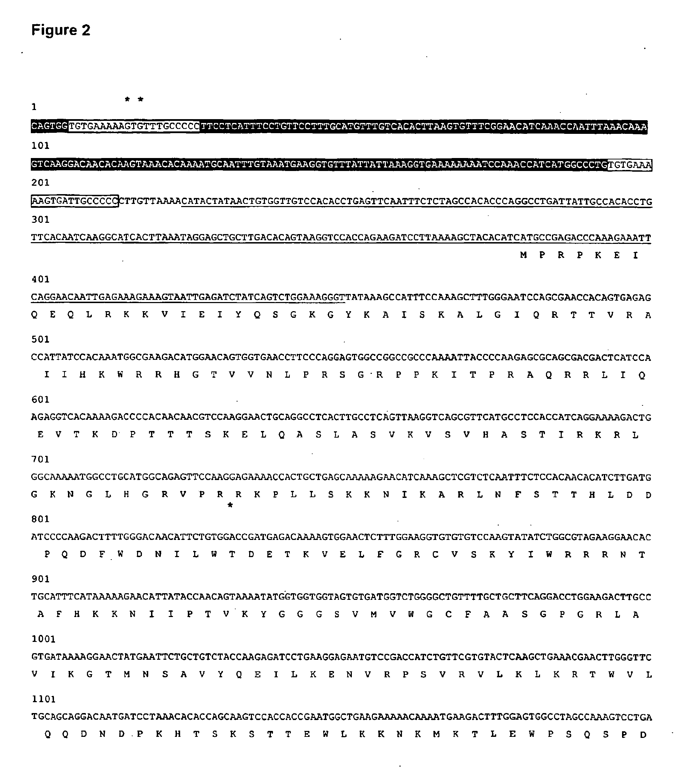

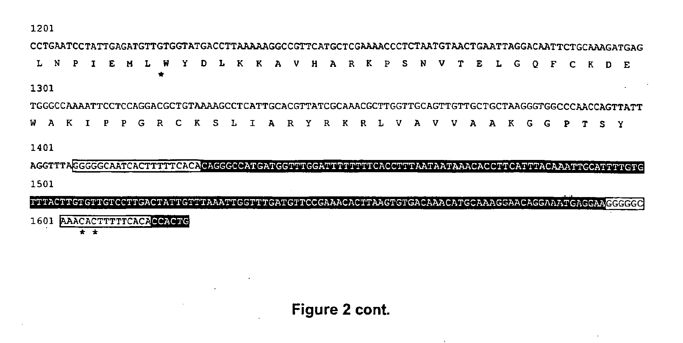

Preferably, the DNA-binding domain comprises or consists of the sequence of SEQ ID NO: 6. Further preferred is, in this and all following embodiments of the present invention, that the transposon comprises inverted repeats wherein the 5' repeat starts at the 5'-end with position No. 1 of Figure 2 and ends at position 214 and the 3' repeat starts at the 5'-end at position 1408 and ends at position 1621 of Figure 2. Also preferably, the inverted repeats and/or the N-terminus of the transposase have a degree of identity with SEQ ID NO: 2 and the inverted repeats, respectively, the shorter fragment of SEQ ID NO: 2 and its inverted repeat referred to herein above, and SEQ ID NO: 3 and/or 4 or SEQ ID NO: 6 of at least 95% such as at least 98%, more preferred at least 99% and most preferred 100%. Identity of nucleic acid sequences (as well as amino acid sequences) can be determined according to conventionally obtained methods or computer programs including BLAST (Altschul, S. F., Gish, W., Miller, W., Myers, E. W. & Lipman, D. J. (1990), J. Mol. Biol. 215:403 - 410) or variants thereof (Altschul, S. F. and Gish, W. (1996), Methods Enzymol. 266, 460 - 480), FASTA (W. R. Pearson and D. J. Lipman (1998), Proc. Natl. Acad. Sci. USA, 85:2444 - 2448) or implementations of the Smith-Waterman algorithm (Smith TF, Waterman MS (1981), J Mol Biol: 147:195 - 7). The term "inverted repeat" has the same meaning as in the art.

The term "transposon-based DNA integration system", including the meaning "gene transfer system" refers to a system that provides components necessary to mediate integration into DNA such as chromosomal DNA. These components are derived, in accordance with the present invention from the *Frog Prince* transposon, the identification of which is described in the appended examples, and further defined in features (a), (b) and (c) of the main embodiment of the invention above. The integration achieved in accordance with the present invention is typical of a class II transposition event. The integration achieved in accordance with the present invention is usually a random event, but may also be effected in a targeted fashion if the transposase is linked, for example, to a DNA targeting domain or interacts specifically with a protein (e. g. via a protein interaction domain attached via a linker) that comprises a DNA targeting domain specifically recognizing a certain DNA sequence; see EP 03 00 2637.1, EP 03 00 2630.6 and EP 03 00 2638.9. The contents of these patent applications is specifically incorporated in its entirety into the present specification by reference. The transposase of the present invention also comprises a nuclear localisation signal (NLS), further sequences of the DNA-binding domain in addition to the helix-turn-helix motifs of SEQ ID: 3 and 4, as well as a catalytic domain.

The components (a) and (b) and/or (c) are preferably present in the system as distinct components. It is further preferred in some cases that at least the transposon is retained as a distinct component. The term "as distinct components" refers to the fact that the components, i.e. the transposon, polynucleotides and/or transposase recited in the system of the invention are physically distinct molecular entities. For example, the transposon recited in (a) and the polynucleotide recited in (c) may not form one single polynucleotide but are present as two distinct polynucleotides that are, optionally separately propagated, in the DNA integration system of the invention.

The term "transposon which is devoid of a polynucleotide encoding a functional transposase" refers to a transposon based DNA molecule no longer comprising the complete sequence encoding a functional, preferably a naturally occurring transposase. Preferably, the complete sequence encoding a functional, preferably a naturally occurring transposase or a portion thereof is deleted from the transposon. Alternatively, the gene encoding the transposase is mutated such that a naturally occurring transposase or a fragment or derivative thereof having the function of a transposase, i.e. mediating the insertion of a transposon into a DNA target site is no longer contained. Alternatively, the activity is significantly reduced such as to at least 50%, better at least 80%, 90%, 95% or 99%. Mutation as referred to above includes substitution, duplication, inversion etc. as described in standard textbooks of molecular biology such as "Molecular Biology of the Gene" (eds. Watson et al.,) 4th edition, The Benjamin/Cummings Publishing Company, Inc., Menlo Park, CA, 1987. The transposon must retain sequences that are required for mobilization by the transposase provided in trans. These are the terminal inverted repeats containing the binding sites for the transposase.

The term "polynucleotide" in accordance with the invention refers to any type of polynucleotide including RNA, DNA or PNA or modifications thereof. Preferred in accordance with the invention is that said term denotes DNA molecules.

The term "having a degree of identity with the repeats within SEQ ID NO: 2 and its inverted repeat, respectively," refers to the fact that SEQ ID NO: 2 denotes/comprises the repeat sequence at the 5'-end of the transposon. The degree of identity applies to the total of sequences of the inverted repeats at the 5' and 3' ends of the transposon (e.g. positions 1 to 124 and 1408-1621 of Fig. 2). The reverse complementary sequence (the inverted repeat) can be found at the 3'-end of the transposon. In accordance with the invention, the 5'-repeat and the 3'-repeat are retained within the transposon comprising the polynucleotide of interest. For further guidance, it is referred to Figure 2, where the repeats are highlighted. The inverted repeats comprised in the transposon-based DNA integration system of the invention have thus, as regards the 5' repeat, a degree of identity of at least 90 % with the repeats comprised in SEQ ID NO: 2 (or the sequence starting at position 1 and ending at position 214 of Figure 2) and, as regards the 3' repeat, a degree of identity of at least 90 % with the repeats comprised in the reverse complementary sequence of SEQ ID NO: 2 (or the sequence starting at position 1408 and ending at position 1621 of Fig. 2), also highlighted at the 3'-region of Figure 2.

The term "a transposase having at its N-terminus a DNA binding domain comprising the sequences of SEQ ID NO: 3 and 4 or having a degree of identity of at least 90 % to SEQ ID NO: 3 and/or 4" refers to a (poly)peptide having transposase function that corresponds regarding the substrate specificity and DNA binding/integration capacity to that of the *Frog Prince* transposase of SEQ ID NO: 1. SEQ ID NO: 3 and 4 correspond to two helix-turn-helix motifs found in the DNA-binding region. It thus also refers to fragments derived from the naturally occurring transposase which lack amino acids preferably within the naturally occurring transposase and which still mediate DNA insertion of the substrate. Alternatively, this term refers to derivatives of the naturally occurring transposase such as fusion proteins comprising the naturally occurring transposase wherein one or more amino acids have been exchanged, deleted, added, or less preferred, where inversions or duplications have occurred. Such modifications are preferably effected by recombinant DNA technology. Further modifications may also be effected by applying chemical alterations to the transposase protein. Said protein (as well as fragments or derivatives thereof) may be recombinantly produced and yet may retain identical or essentially identical features as the naturally occurring protein.

The term "(poly)peptide" refers alternatively to peptides or to polypeptides. Peptides conventionally are amino acid sequences having up to 30 amino acid whereas polypeptides (also referred to as "proteins") comprise stretches of at least 31 amino acids.

In accordance with the present invention, a novel transposable element system was developed from inactive elements found in the genome of the amphibian species *Rana pipiens.* This new system, that is named *Frog Prince* (FP), may be conveniently be used as a vector for the insertion of genetic material into the chromosomes of vertebrate cells.

Some of the main characteristics of a desirable transposon vector are: ease of use, relatively wide host range, efficient chromosomal integration, and stable maintenance of faithful transgene expression throughout multiple generations of transgenic cells and organisms.

More specifically, the advantages of the transposon-based DNA integration system of the present invention in particular for gene transfer in vertebrates over the available prior art systems may be described as follows:
It can transform a wide range of vertebrate cells; further, because it is a DNA-based transposon, there is no need for reverse transcription of the polynucleotide of interest and preferably the transgene, which introduces mutations in retroviral vector stocks. Since transposons are not infectious, transposon-based vectors are not replication-competent, therefore do not spread to other cell population. In addition, the transposon-based DNA integration system of the invention requires only about 230 bp transposon inverted repeat DNA flanking a polynucleotide of interest such as a transgene on each side for mobilization; transposition is inducible, and requires only the transposase protein, thus one can simply control the site and moment of jumping by control of transposase expression. Further, the transposon-based DNA integration system of the present invention mediates stable, single-copy integration of genes into chromosomes which forms the basis of long-term expression throughout multiple generations of transgenic cells and organisms. Once integrated, the elements are expected to behave as stable, dominant genetic determinants in the genomes of transformed cells, because
   1) the presence of the transposase is only transitory in cells and is limited to a time window when transposition is catalyzed, and 2) there is no evidence of an endogenous transposase source in vertebrate cells that could activate and mobilize the integrated elements; with the exception of some frog species, there are no endogenous sequences in vertebrate genomes with sufficient homology to the transposon-based DNA integration system of the invention that would allow recombination and release of transpositionally competent (autonomous) elements. The system of the present invention also has a further advantageous feature compared to many prior art transposons as it is less sensitive to a phenomenon called overexpression inhibition. Namely, even by increasing the transposase concentration of the system of the present invention over a certain limit, the transposase still does not inhibit the transposition reaction. This feature of prior art transposons limiting certain ranges of the transposition reaction may be disadvantageous for some applications, such as certain applications of gene therapy. The system of the invention can be overloaded by two magnitudes of higher amount of transposase without experiencing the phenomenon of overexpression inhibition.

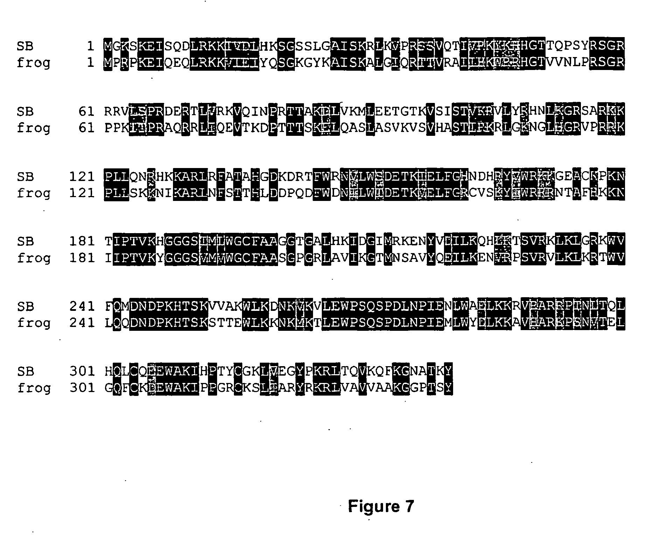

For a comparison of the amino acid sequences of the transposases of SB and Frog Prince, please be referred to Figure 7.

Another important advantage of the transposon-based DNA integration system of the present invention is that the transposase specifically interacts or primarily interacts with the substrate only, i.e. with the inverted repeats recited in feature (a) of the main embodiment of the invention. The significance of this is that the transposase cannot interact with and mobilize transposable elements that are divergent from the Frog Prince transposon and are endogenous in genomes (e.g. the human or zebrafish genomes). And vice versa, endogenous transposases divergent from the Frog Prince transposase will not mobilize Frog Prince transposons. This specificity of transposon-transposase interaction ensures stability of integrated transgenes and stability of endogenous genes.

In a preferred embodiment of the transposon-based DNA integration system of the invention, the transposase comprises a bipartite nuclear localisation signal represented by SEQ ID NO: 5, and/or the DNA-binding domain of SEQ ID NO: 6 and/or a catalytic domain with a DD(34)E signature represented by SEQ ID NO: 7. Alternatively, the nuclear localisation signal, the catalytic domain and/or the DNA-binding domain have a region of identity of at least 90 %, preferably of at least 95 %, more preferred of at least 98 % and most preferred of at least 99 % with sequences represented by SEQ ID NOs: 5, 6 and/or 7.

As with the main embodiment of the invention, molecular modifications of the wild type sequence are feasible for this preferred embodiment as long as the above recited activity is maintained.

Particularly preferred is in accordance with the present invention that in the transposon-based DNA integration system said transposase has the amino acid sequence having a degree of identity of at least 90 %, preferably of at least 95 %, more preferred of at least 98 % and most preferred of at least 99 % with SEQ ID NO: 1. In another most preferred embodiment, the transposase has an amino acid sequence that is identical with that of SEQ ID NO: 1.
The embodiment of the invention relating to the 100 % identity pertains to the wild type *Frog Prince* transposase as described in the appended examples. The transposon-based DNA integration system of the invention in a further preferred embodiment requires that the polynucleotide of interest is a gene.
The term "gene" is well understood in the art and comprises a transcribed portion as well as 5' and 3' flanking regions, including a promotor (see, e.g. "Biotechnology" 2nd edition 1993, page 666 et seq., ed H.-J. Rehm and G. Reed, VCH, Weinheim). The term gene as used in accordance with the invention includes also artificial genes wherein, for example, the transcribed seqeunce is under the control of a heterologous promotor and, optionally, also associated with additional heterologous control elements such as a heterologous enhancer. The gene of interest may encode markers such as the green fluorescent protein for in vivo monitoring and reporters such as luciferase or antibiotic resistance genes.

The polynucleotide of interest and preferably gene may be any naturally occurring or artificial polynucleotide or gene such as a gene derived from bacteria, viruses, bacteriophages or animals. It is preferred in accordance with the invention that said gene is derived from a mammal, fish, amphibian such as frog, reptile or bird. Particularly preferred is that said mammal is a human.

The polynucleotide of interest may be of a variety of natures. For example, it may be of non-coding nature and thus be useful in the targeted disruption of a gene that, upon overexpression, is involved in the etiology of a disease. In a further example, the transposon could contain promoter sequences that activate gene expression if the transposon inserts sufficiently close to an endogenous gene. Moreover, the transposon might lack any sequence in addition to the sequences that are required for transposition, in case a suitable selection scheme is available (e.g. one based on altered cellular phenotypes) to identify insertions into particular targets. In a further alternative, the polynucleotide of interest serves as a sequence tag that can subsequently be used to identify the transposon insertion.

In another preferred embodiment of the transposon-based DNA integration system of the invention, said polynucleotide of interest encodes a therapeutically active (poly)peptide.
In this embodiment, (poly)peptides of therapeutic value may be targeted into cells in need of such (poly)peptides. If tissue-specific expression is desired, the tissue-specific promoters may drive expression of said (poly)peptides. The therapeutically active (poly)peptide may be any peptide or protein that counteracts the onset or progression of a disease. It may directly or indirectly interfere with said onset or progression. Therapeutically active (poly)peptides include those of the class of growth factors or differentiation factors such as GCSF, GM-CSF, as well as interleukins and interferons or engineered antibody derivatives such as scFvs that bind to an adverse compound within the body. The transposon-based system could be used as a vector for gene therapy for monogenic diseases such as haemophilia. cDNAs, equipped with suitable transcriptional regulatory sequences, encoding blood clotting factors FactorVIII or FactorIX could be incorporated in the transposable element vector. Transposase mediates stable integration of the therapeutic genes into chromosomes, ensuring long term gene expression and an increase in of transgene products in the serum.

The transposon-based DNA integration system in another preferred embodiment of the invention requires that said polynucleotide of interest transcribes into siRNA and encodes a selectable marker.

The polynucleotide may be transcribed into hairpin RNA molecules that mediate RNAi with regard to the expression of a desired target; see, for further guidance, for example, Elbashir et al., Nature 411 (2001), 494-498, Bernstein et al., RNA 7 (2001), 1509-1521, Boutla et al., Curr. Biol. 11 (2001), 1776-1780. The transposon-based system of the present invention may be used for stable insertion of RNAi expression cessettes into chromosomes, for stable gene knock down in vertebrate cells. The embodiment requires that the transposon contains both a selectable marker such as an antibiotic marker gene and the RNAi expression cassette. Transformant cells can be selected on the basis of e. g. antibiotic resistance, and those cells will likely express the RNAi cassette efficiently. This method will simplify the generation and screening of stable RNAi clones by transposition.

In a further preferred embodiment of the present invention, in the transposon-based DNA integration system said polynucleotide of interest comprises a intron splice acceptor sitea selectable marker gene wherein said gene lacks the methionine start codon and contains a polyA addition signal to stabilize the mRNA. This embodiment can be used as a gene trap expression cassette inside the transposon vector to generate and select for transposition events in expressed genes. For example, the gene trap transposon may carry the mouse engrailed 2 intron containing a splice acceptor site and a selectable marker such as the neomycin resistance marker gene that lacks an initiator methionine codon. Therefore, expression of the selectable marker can only be obtained if a fusion protein with an endogenous gene is made. The gene trap transposon construct may be transfected together with a transposase source into cells such as human HeLa cells. Some of the gene trap transposons insert into the introns and untranslated regions of genes, a fraction of which will be in the proper orientation so that a fusion transcript between the endogenous gene and the (as an example) neo marker gene can be produced. Several transposon insertions were cloned from G418-resistant cell clones. All of these insertions occurred in genes. Integration of the gene trap vector into actively transcribed genes can act as a locus specific marker in living animals and will provide tags for identifying the disrupted genes.

The transposon-based DNA integration system in another preferred embodiment requires that said transposase has an enhanced transposase activity. Point mutant versions of proteins, carrying slight modifications in their coding sequence often show higher activities compared to the original versions. For this reason, several proteins were modified in order to obtain "hyperactive" versions. A well known example is the GFP (Green Fluorescence Protein) vs eGFP protein (e- enhanced) that was modified to the naturally occurring protein and have better characteristics for laboratory use. Hyperactive transposases are also known from bacteria (Tn5) and insects (Himar1). These transposases are reported to support DNA transposition at 1-2 orders of magnitude higher level compared to the original protein.

Facing the high expectation toward the performance of vertebrate transposons, a HYPERACTIVE SCREEN on the Frog Prince transposase was initiated. This experiment is expected to produce a system that performs 10-100-fold better than the original.

In the appended examples it is demonstrated that the attempt to produce a "hyperactive transposon system" is a successful approach.

In an additional preferred embodiment of the transposon-based DNA integration system of the present invention, the transposon of (a) and/or the polynucleotide of (c) is comprised in at least one vector, i.e. in one or more vectors (alternatively, the transposon may be provided without vector sequences, e.g., in circularised form).
The vector employed for any of the above recited polynucleotides may, in accordance with the present invention be an expression, a gene transfer or gene targeting vector. Expression vectors are well known in the art and widely available; see Ausubel et al., *loc. cit.* In this more preferred embodiment of the vector of the invention the polynucleotide is operatively linked to expression control sequences allowing expression in prokaryotic or eukaryotic cells or isolated fractions thereof.
Expression of said polynucleotide(s) comprises transcription of the polynucleotide, preferably into a translatable mRNA. Insofar, the source of the transposon may be mRNA. In an alternative embodiment, transposase mRNA is introduced into a cell of interest. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E. coli*, and examples for regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL).

Gene therapy, which is based on introducing therapeutic genes into cells by ex-vivo or in-vivo techniques is one of the most important applications of gene transfer. Suitable vectors, methods or gene-delivering systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Onodua, Blood 91 (1998), 30-36; Verzeletti, Hum. Gene Ther. 9 (1998), 2243-2251; Verma, Nature 389 (1997), 239-242; Anderson, Nature 392 (Supp. 1998), 25-30; Wang, Gene Therapy 4 (1997), 393-400; Wang, Nature Medicine 2 (1996), 714-716; WO 94/29469; WO 97/00957; US 5,580,859; US 5,589,466; US 4,394,448 or Schaper, Current Opinion in Biotechnology 7 (1996), 635-640, and references cited therein. In particular, said vectors and/or gene delivery systems are also described in gene therapy approaches e.g. in neurological tissue/cells (see, inter alia Blömer, J. Virology 71 (1997) 6641-6649) or in the hypothalamus (see, inter alia, Geddes, Front Neuroendocrinol. 20 (1999), 296-316 or Geddes, Nat. Med. 3 (1997), 1402-1404). Further suitable gene therapy constructs for use in neurological cells/tissues are known in the art, for example in Meier (1999), J. Neuropathol. Exp. Neurol. 58, 1099-1110. The vectors used in accordance with the invention may be designed for direct introduction or for introduction via liposomes, or viral vectors (e.g. adenoviral, retroviral), for electroporation, ballistic (e.g. gene gun) or other delivery systems into the cell. Additionally, a baculoviral system can be used as eukaryotic expression system for the nucleic acid molecules of the invention. The introduction and gene therapeutic approach should, preferably, lead to the expression of a functional molecule, preferably a therapeutically active molecule, whereby said expressed molecule is particularly useful in the treatment, amelioration and/or prevention of any disease that may be ameliorated, prevented or treated by gene therapy approaches.

In a particularly preferred embodiment, at least one of said vectors is a plasmid.
Plasmids are well known in the art and described for recombinant purposes, for example, in Sambrook et al, "Molecular Cloning, A Laboratory Manual", 2nd edition, CSH Press, Cold Spring Harbor, 1989; Ausubel et al., "Current Protocols In Molecular Biology" (2001), John Wiley & Sons; N.Y. They are characterized as small extrachromosomal, usually circular double-stranded DNA molecules that replicate autonomously. They naturally occur in prokaryotes as well as eukaryotes and usually comprise at least one origin of replication and a low number of genes. The form of a plasmid-based vector is particularly advantageous since plasmid production is easy, inexpensive, and can be scaled up.

The present invention also relates to a host cell transfected or transformed with the transposon-based DNA integration system of the invention.
The host cell of the invention may be a prokaryotic cell but is preferably a eukaryotic cell such as an insect cell such as a Spodoptera frugiperda cell, a yeast cell such as a Saccharomyces cerevisiae or Pichia pastoris cell, a fungal cell such as an Aspergillus cell or a vertebrate cell. In the latter regard, it is preferred that the cell is a mammalian cell such as a human cell. The cell may be a part of a cell line.

Additionally, the present invention relates to a non-human transgenic animal comprising the transposon of (a) or the transposon-based DNA integration system of the present invention wherein at least one of the components is comprised in a vector or in circularized form stably integrated into its genome.

The transgenic animal may be any animal available to manipulation and and wherein manipulation results in a transgenic animal. It is preferably a mammal such as a rat or a mouse. On the other hand, it may be a fish such as a zebrafish or an amphibian such as a frog.

In accordance with the above, the invention also relates to the use of the transposon-based DNA integration system of the invention for the preparation of a medicament for a transfer of a polynucleotide of interest into cells of a vertebrate by introducing the transposon-based DNA integration system of the invention into said cells.

Preferably, the transfer is effected in vitro.

In a different preferred embodiment of the invention, said transfer is effected in vivo.

More preferred in accordance with the present invention is the use whereby
(a) cells are selected wherein the polynucleotide is stably integrated into the chromosomes of said cells; and whereby
(b) said cells or cells derived from said cells are introduced or reintroduced into a vertebrate of the same species.

Selection of cells wherein the polynucleotide is stably integrated into the chromosomes can be done by physically showing covalent linkage of the transposon and chromosomal DNA (e.g. by PCR using the transposon sequence as a unique sequence tag).

In a particularly preferred embodiment of the use of the invention, the vertebrate into which said cells are reintroduced is the vertebrate from which the cells were taken prior to said transfer.

A variety of techniques are known in the art for effecting said transfer. Consequently, there are a number of different methods to introduce the components of the transposon system into cells. Because naked plasmid DNA usually is not efficiently taken up by cells, it is advantageously associated or coupled with other molecules that can penetrate cell membranes. Gene guns either use small particles coated with DNA that are shot into cells thereby delivering their cargo, or use high speed solvent (DNA solution) delivery into living cells. The common principle is the physical delivery of DNA into cells via high speed penetration through cellular membranes. Liposomes are used to package DNA into small artificial membrane particles, that can either fuse with the cell membrane, or taken up by the cell via endocytosis, thereby internalizing the DNA cargo. Electroporation uses high voltage electric shock to transfer DNA across cell membranes. Adenovirus-polylysine complexes are used to transfer DNA via the natural ability of the adenovirus to infect cells. These various methods are described in Current Protocols in Human Genetics (1997). Preferred in accordance with the present invention are methods wherein said transfer is effected by means of a gene gun, or by means of a gene transfer mediated or assisted by liposomes, polyethyleneimine, adenovirus-polylysine-DNA complexes, lipofection, electroporation, transfection or infection/transduction.

As regards the option of using infection, it is preferred that said infection or transduction is mediated or assisted by recombinant retrovirus, recombinant adenovirus, recombinant herpes virus or recombinant adeno-associated virus.

In a different preferred embodiment of the use of the present invention, said cells are somatic cells. In conjunction with the manipulation of somatic cells, it is particularly preferred that the vertebrate is a mammal, fish, amphibian, reptile or bird. Most preferred is that said mammal is a human.

In an alternative preferred embodiment of the use of the present invention, said cells are non-human germ line cells. In conjunction with the manipulation of germ line cells, it is particularly preferred that the vertebrate is a non-human mammal, fish, amphibian, reptile or bird.
In the connection with non-human germ line cells; it is preferred that said cells are pluripotent or multipotent. The corresponding use in a different preferred embodiment further encompasses, after reintroducing said non-human germ line cells or a cell derived from said non-human germ line cell into a vertebrate of the same species, growing of a transgenic vertebrate wherein said vertebrate or transgenic vertebrate is not a human.

The genenation of transgenic animals has been briefly referred to herein above and is a technology well established in the art.
A method for the production of a transgenic non-human animal, for example transgenic mouse, is provided by the use of a polynucleotide or targeting vector as above which is introduced into a non-human germ cell, an embryonic cell, stem cell or an egg or a cell derived therefrom. The non-human animal can be used in accordance with a screening method of the invention described herein. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994)). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born e.g. as chimeric mice. The resultant transgenic mice are chimeric for cells having either the recombinase or reporter loci and are backcrossed and screened for the presence of the correctly targeted transgene (s) by PCR or Southern blot analysis on tail biopsy DNA of offspring so as to identify transgenic mice heterozygous for either the recombinase or reporter locus/loci.

Methods for producing transgenic flies, such as Drosophila melanogaster are also described in the art, see for example US-A-4,670,388, Brand & Perrimon, Development (1993) 118: 401-415; and Phelps & Brand, Methods (April 1998) 14: 367-379. Transgenic worms such as C. elegans can be generated as described in Mello, et al., (1991) Efficient gene transfer in C.elegans: extrachromosomal maintenance and integration of transforming sequences. Embo J 10, 3959-70, Plasterk, (1995) Reverse genetics: from gene sequence to mutant worm. Methods Cell Biol 48, 59-80.
All the applications that have been herein before discussed with regard to a transgenic animal also apply to animals carrying two, three or more transgenes. It might be also desirable to inactivate protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the encoding RNA. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similarly, the expression of the mutant protein may be controlled by such regulatory elements.

Furthermore, in the transgenic animals thus generated which cells contain (preferably stably integrated into their genome) the transcription and/or expression of the nucleic acid molecule or part thereof, one may reduce the synthesis of a desired protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product.
Techniques how to achieve this are well known to the person skilled in the art. These include, for example, the expression of antisense-RNA, ribozymes, siRNA, molecules which combine antisense and ribozyme functions and/or of molecules which provide for a co-suppression effect; see also supra. When using the antisense approach for reduction of the amount of desired proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a desired protein. In this case the homology is preferably higher than 80%, particularly higher than 90% and still more preferably higher than 95%. The reduction of the synthesis of desired protein in the transgenic eukaryotic cells can result in an alteration in, e.g., calcium signaling. In transgenic animals comprising such cells this can lead to various physiological, developmental and/or morphological changes.

If non-human germ cells or cells derived from non-human germ line cells such as cells in the blastocyst stage are manipulated, it is preferred that said introduction or reintroduction is mediated or effected by sperm, microinjection or by means of a gene gun.

Additionally, the present invention relates to an in-vitro method of effecting RNAi comprising
(a) stably introducing the transposon being part of the transposon-based DNA integration system of the invention wherein said polynucleotide of interest transcribes into siRNA and encodes a selectable marker into a cell;
(b) selecting for cells expressing the selectable marker; and
(c) assessing whether the transcription/translation of the desired gene is effected by RNAi.

The applications of this method of the invention have been discussed in connection with the corresponding embodiment of the transposon-based DNA integration system of the invention herein above. These applications as well as the preferred embodiments descibed in connection with the DNA-integration system of the present invention *mutatis mutandis* apply to the method of the invention. Integration of the transposon is mediated by the transposase referred to in this specification which preferably is introduced as a protein or a nucleic acid encoding the protein into the cell or a progenitor of the cell.

The present invention further relates to an in-vitro method of gene trapping genes comprising
(a) introducing the transposon-based DNA integration system of the invention wherein said polynucleotide of interest comprises a intron splice acceptor site, a selectable marker gene wherein said gene lacks the methionine start codon and contains a polyA addition -signal into a cell; and
(b) assessing for the expression of a selectable marker wherein expression of a selectable marker is indicative of integration of the transposon into a transcibed gene of the cell.
As with the previous embodiment, the applications of this method of the invention have been discussed in connection with the corresponding embodiment of the transposon-based DNA integration system of the invention herein above. These applications as well as the preferred embodiments descibed in connection with the DNA-integration system of the present invention *mutatis mutandis* apply to the method of the invention. These applications also *mutatis mutandis* apply to the method of the invention. In addition to the splice acceptor site, portions of the intron or the complete intron may be contained in the polynucleotide of interest.

In a preferred embodiment of the method of the invention, said in-vitro method further comprises
(c) identifying the disrupted gene by means of the integrated transposon as a tag. As stated above, integration of the gene trap vector into actively transcribed genes can act as a locus specific marker in living animals and will provide tags for identifying the disrupted genes.

The invention also relates to a transposon characterized in that it comprises or consists of inverted repeats having a degree of identity with the repeats within SEQ ID NO: 2 and the inverted repeat of SEQ ID NO: 2, respectively, of at least 90%; and a transposase having at its N-terminus a DNA binding domain comprising the sequences of SEQ ID NO: 3 and 4, or more preferred, the binding domain of SEQ ID NO: 6. More preferred is a transposon wherein the transposase has the amino acid sequence of SEQ ID NO: 1 and the flanking sequences (repeats) comprise or consist of the sequences of SEQ ID NO: 2. In further alternative embodiments, amino acid and nucleic acid sequences are employed which are to at least 90 %, preferably of at least 95 %, more preferred of at least 98 % and most preferred of at least 99 % identical to the sequences of SEQ ID NOS: 1 (or 3 and/or 4 or 6) and 2.

Finally, the present invention relates to a composition comprising the the transposon-based DNA integration system of the invention. The composition may, e.g., be a diagnostic composition or a kit or a pharmaceutical composition. The various components of the composition may be packaged in one or more containers such as one or more vials. The vials may, in addition to the components, comprise preservatives or buffers for storage.

Preferably, the composition is a pharmaceutical composition.
The pharmaceutical composition composition may be in solid, liquid or gaseous form and may be, inter alia, in a form of (a) powder(s), (a) tablet(s), (a) solution(s) or (an) aerosol(s). Said composition may comprise at least two, preferably three, more preferably four, most preferably five sets of the distinct components referred to above of the invention.

It is preferred that said pharmaceutical composition, optionally comprises a pharmaceutically acceptable carrier, excipient and/or diluent. The herein disclosed pharmaceutical composition may be particularly useful for the treatment of any disease that can be prevented, alleviated or cured by means of gene therapy. Said disorders comprise, but are not limited to haemophilia, deficiency in alpha-antitrypsin, familiar hypercholesterolemia, muscular dystrophy, cystic fibrosis, cancer, severe combined immunodeficiency, and diabetes.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in muscle, liver, lung, pancreas, or solid tumors. The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute. A preferred dosage for the administration of DNA is 10⁶ to 10¹² copies of the DNA molecule.
Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition. It is particularly preferred that said pharmaceutical composition comprises further agents like immune enhancers etc.

The figures show:

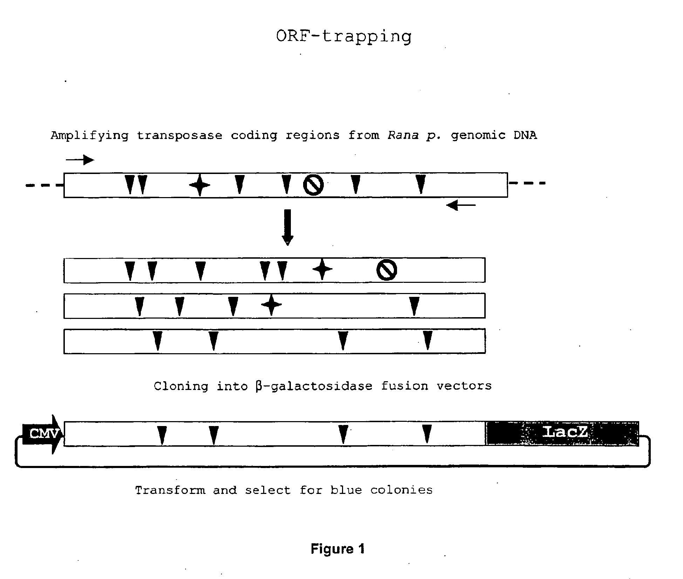

**Figure 1****. Strategy for trapping transposase ORFs from the *Rana pipiens* genome.** Transposase genes are PCR-amplified from genomic DNA, using primers (indicated by arrows) designed to flank the predicted gene sequences. A collection of transposase coding regions (represented by empty boxes) can be amplified. The vast majority of these genes is defective due to point mutations (▼), frameshifts (◆) and premature translational stop codons (•). Uninterrupted ORFs can be selected by cloning the PCR products in fusion with the LacZ gene driven by the CMV promoter, transformation into *E. coli,* and plating on X-gal plates.
**Figure 2****. Deduced consensus sequence of the full-length *Frog Prince* transposable element.** The IRs are displayed in a black background. The 21 bp long DRs are indicated in white boxes. The encoded amino acid sequence of the transposase is displayed below the DNA sequence. The newly identified sequences that are missing from the *Xenopus* Txr consensus are underlined. The asterisks indicate base pair positions where sequences between the *Rana* and *Xenopus* elements are different within the transposase binding sites, and where replacements were introduced in the transposase gene in order to derive the consensus sequence.

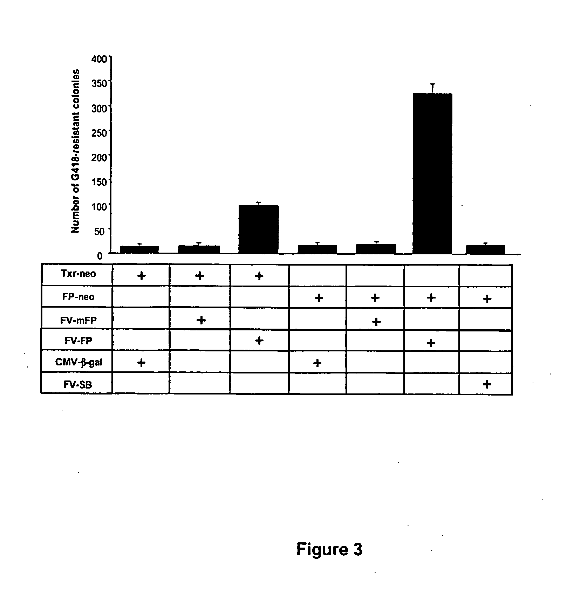

**Figure 3****. Transposition and substrate recognition of FP in human HeLa cells.** Different combinations of donor and helper plasmids indicated in the table were cotransfected into HeLa cells. Transfection of pCMV-βgal with the donor plasmids served as control. The efficiency of transgene integration was estimated by counting antibiotic-resistant colonies, after plating cells under G418 selection. The numbers on the left represent the mean values of the numbers of colonies per 10⁵ cells plated. Results represent at least three transfection experiments. The error bars indicate SEM.

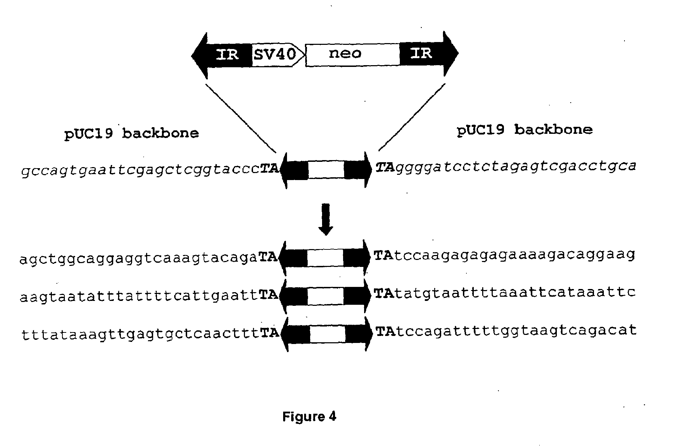

**Figure 4****. FP-mediated cut-and-paste transposition into human chromosomes.** On top, a schematic of the *FP*-neo element is shown. IRs are represented by black arrows, the SV40 promoter and the neomycin-resistance marker gene (neo) are indicated in the white arrow and box, respectively. Neighboring pUC19 vector backbone sequences that flank the element in the donor construct are shown in italics. Three regions of human genomic sequences that served as target sites for the transposase are illustrated below. Precise cut-and-paste transposition led to the duplication of the genomic TA target dinucleotides, that are typed in bold.

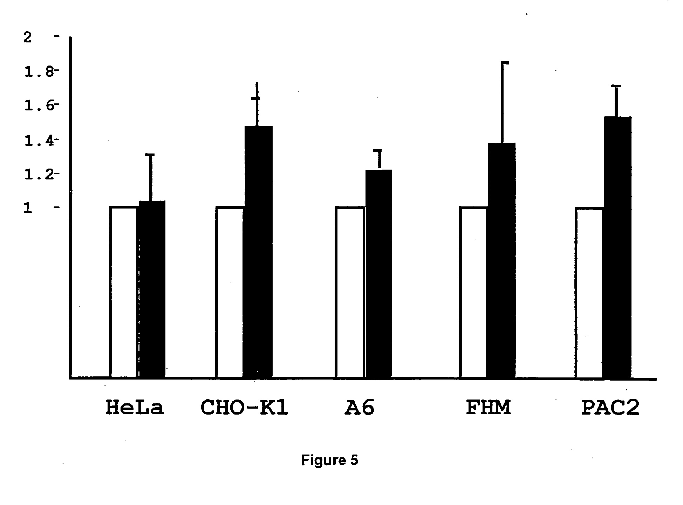

**Figure 5****. Activity of FP in diverse vertebrate species.** The donor and helper plasmids of FP and SB were cotransfected in Hela (human), CHO-K1 (hamster), A6 *(Xenopus laevis),* FHM (fathead minnow) and PAC2 (zebrafish) cell lines. Transposition efficiencies were calculated by deriving ratios between the numbers of G418-resistant cell clones obtained in the presence *versus* in the absence of the transposases. Activities of *FP* (indicated by black columns) were compared to those of *SB* (white columns). Relative efficiencies are indicated on the *y*-axis, the activity of *SB* in a given cell line is arbitrarily set to value 1. The numbers shown are mean values obtained by at least three repetitions of transfections. The error bars show SEM.

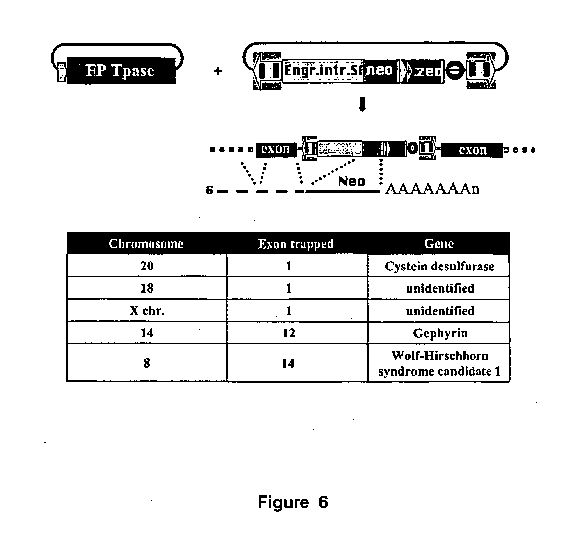

**Figure 6****. Gene trap construct based on the Frog Prince transposon and results obtained with the gene trapping method.** The figure shows the crucial components of the gene trap vector based on the Frog Prince transposon. For further details, see appended Example 6. Also shown are the genes and exons trapped with the method of the invention including the chromosomes on which the genes are located.
**Figure 7****. Alignment of Sleeping Beauty and Frog Prince transposase amino acid sequences**. Identical amino acids are in a black background and conserved amino acids are in a grey background. The level of identity of the two transposase amino acid sequences is about 50%.

The examples illustrate the invention.

### Example 1: Isolation of transposase genes from Rana pipiens with ORF-trapping.

All naturally occurring Tc1/*mariner* elements isolated to date from vertebrate species are inactive (nonautonomous) copies containing numerous mutations in their transposase genes. Relatively high copy number of these elements in genomes is practically prohibitive to the isolation of functional transposase genes using nonselective methods. In search for potentially active transposase genes in vertebrates, we devised an open reading frame (ORF)-trapping method. The procedure is based on generating a pool of PCR products from genomic DNA, using primers flanking the transposase gene sequences (Fig. 1). The 5'-primer contains the predicted translational initiation signal, the 3'-primer lacks the stop codon. The PCR product is then cloned into an expression vector to generate fusion genes with lacZ. The recombinant plasmids are transformed into *E. coli,* and plated on X-gal plates. Blue colonies can only arise if the cloned sequences are in frame with the lacZ gene.

The method should be useful for the selection of uninterrupted ORFs, but it does not filter out missense mutations or in-frame insertions or deletions.

We applied the ORF-trap on genomic DNA samples from *Rana pipiens,* using PCR primers designed to the published consensus sequence of Txr elements in *Xenopus laevis* (Lam et al., 1996). After transforming the library into bacteria, four blue colonies (out of approximately 100 colonies in total) indicated the presence of transposase-coding sequences that did not contain premature stop codons. All of the four cloned sequences were longer than the predicted consensus Txr transposase gene. The *Rana* sequences contained a region containing part of the 5'-terminal inverted repeat followed by sequences that were missing in the Txr copies. Apparently, Txr elements contain a conserved deletion of 180 bps covering the N-terminal part of the transposase gene, leading to a false transposase sequence prediction by Lam et al. (1996). Similarly, a conserved deletion has been described in the case of *Tdr1* elements in zebrafish (Izsvak et al., 1995). The rest of the *Rana* sequences and Txr coding regions showed approximately 90% similarity (data not shown).

To check the efficacy of our ORF-trapping, the genomic copy number of the *Rana* transposon was estimated by dot blotting. Assuming that the size of the *R. pipiens* haploid genome is 6,6x10⁹ bps, we estimated that the transposase gene is represented 8000 times per haploid genome, which represent approximately 0, 1 % of the *R. pipiens* genome. This number is within the range of from 0.02 to 0.6% found for other *Tc1-*like elements in other species. These data indicate that our novel ORF-trapping method appreciably contributed to the identification of transposase genes that are in a relatively good shape, from a genome where this transposon family is abundantly represented.

### Example 2: Identification of the two components of the Frog Prince transposon system.

The consensus *Rana* gene (Fig. 2) encodes a typical Tc1-like transposase containing a presumptive, N-terminal DNA-binding domain composed of two predicted helix-turn-helix motifs (Plasterk et al., 1999), a bipartite nuclear localization signal (Ivics et al., 1996) and a catalytic domain with the DD(34)E signature (Plasterk et al., 1999) (Fig. 2). One of the three different transposase genes we isolated differed only in two nucleotides from the consensus, resulting in two amino acid substitutions in its ORF. One of them was a Thr→Ser(152) exchange in the first part of the catalytic domain of the transposase. The other missense mutation was due to the deamination of a ^{5m}C residue of a CpG site that led to a Arg→Cys(315) substitution close to the C-terminus of the protein. Site-specific PCR mutagenesis was used to derive the sequence of the consensus *Rana* transposase gene (Fig. 2). The encoded amino acid sequence of the FP transposase is shown in SEQ ID NO: 1.

In order to derive the binding sites for the Rana-type transposase, splinkerette PCR was applied on genomic DNA to amplify the complete inverted repeats together with genomic flanking sequences. Alignments of five different clones revealed 214 bp long, perfect inverted repeats flanking the transposase genes (Fig. 2). The DNA sequences of the inverted repeats are also shown in SEQ ID NO: 2. The *Rana* transposons are typical IR/DR-type elements, i.e. the IRs contain four transposase binding sites, that are represented as directly repeated sequences (DRs) at the ends of the IRs (Ivics et al., 1997). The DRs are 21 nucleotide long, and differ in one nucleotide between the outer and internal sites. The IR sequences together with the consensus transposase gene constitute the components of a novel transposable element system, that we named the *Frog Prince* (Fig. 2).

### Example 3: Transposition of FP.

*Sleeping Beauty* shows high transpositional activity in human cells (Ivics et al., 1997). Therefore, the initial tests for transpositional activity of the *Frog Prince* element were done in cultured HeLa cells, using a transposition assay established for SB (Ivics et al., 1997). The assay is based on cotransfection of a helper plasmid expressing the transposase and the donor construct containing the transposon with a neomycin-resistance (neo) gene between the terminal IRs. The efficiency of transgene integration catalyzed by the transposase was elucidated from the number of G-418-resistant colonies due to chromosomal integration and expression of the selectable marker gene (Ivics et al., 1997). The reconstructed consensus *Rana* transposase ORF (in pFV-*FrogPrince),* and its predecessor gene containing the two amino acid changes (in pFv-m*FrogPrince)* were transfected together with either the Txr-type (pTxr-neo) or with the *FrogPrince-type (*p*FrogPrince*-neo*)* substrate constructs (Fig. 3). A 17-fold increase in colony number was detected when pFV*-FrogPrince* was cotransfected with its own substrate, p*FrogPrince*-neo*.* This result demonstrates that we successfully derived and engineered an active transposon system from the *Rana pipens* genome. The mFrogPrince transposase was completely inactive, indicating the importance of either or both of the amino acids T(152) and R(315). Interestingly, we observed a 5-fold increase in the number of G418-resistant cell colonies when pFV-*FrogPrince* was cotransfected with pTxr-neo (Fig. 3). Thus, the *Rana* transposase can cross-mobilize *Xenopus* transposons, indicating that the two transposon families in these species diverged recently. In contrast, no cross-mobilization was observed between FP and *Sleeping Beauty.* Together the data demonstrate that the *Frog Prince* transposon system can significantly increase the efficiency of transgene integration from plasmids into the human genome.

### Example 4: Cut-and-paste transposition of Frog Prince into the human genome.

Tc1/*mariner* elements transpose via a cut-and-paste mechanism into TA dinucleotides. The TA target dinucleotides are duplicated and flank the integrated transposon on both ends, which is a hallmark of Tc1/*mariner* transposition (Plasterk et al., 1999). In order to examine flanking sequences of the integrated *Prince* transposons, genomic DNA was isolated from individual G418-resistant HeLa clones, and subjected to splinkerette-PCR. We have cloned and sequenced the left and right junctions bordering three different transposon insertions, two of them from introns and one from a non-genic sequence (data not shown). In all three cases the *Prince* insertions were flanked by the expected TA dinucleotides on both sides of the transposons, followed by sequences different from each other and those of the transposon-donor plasmid (Fig. 4). Similar results were obtained with four other flanking sequences bordering either the right, or the left IR of the transposon (data not shown). Inspection of the target sites revealed that all of the insertions occurred at TA dinucleotides in different human chromosomes (Fig. 4). In sum, these data show that FP follows precise cut-and-paste transposition into various locations in the human genome.

### Example 5: FP is active in various vertebrate cell lines.

*SB* has varying transpositional activity in different vertebrate cell lines (Izsvak et al., 2000). However, SB is a synthetic element of fish origin and *Frog Prince* was reconstructed from the genome of an amphibian, thus the same set of cell lines can provide different permissive environments to the two transposon systems. Therefore, we compared the activities of the two systems in cultured cell lines two mammalian, an amphibian and two fish with the standard transposition assay. The plasmids used for cotransfections were pFV-*SB* and pT/neo (Ivics et al., 1997) in the case of *SB*, and pFV-*FrogPrince* and p*FrogPrince*-neo in the case of the *FP* system. The vector backbones, the promoters, the poly-A signals and the transposon marker genes were identical in the constructs of the two systems. FP appears to be more active than SB in all of the cell lines tested (Fig. 5). However, the difference in the transpositional efficiencies of the two transposon systems is modest, and is statistically significant only in the PAC2 zebrafish cell line. These data demonstrate that transposition of FP is not restricted to phylogenetically close taxa, and that it is the most active transposable element in vertebrate species descibed to date.

### Example 6. Gene trapping with Frog Prince in human HeLa cells

We have used a gene trap expression cassette inside the Frog Prince transposon vector to generate and select for transposition events in expressed genes. The gene trap transposon carries the mouse engrailed 2 intron containing a splice acceptor (SA) site and the neomycin resistance marker gene (neo) that lacks an initiator methionine codon. Therefore, neo expression can only be obtained if a fusion protein with an endogenous gene is made. The transposon also contains a zeo expression cassette to select for insertion events anywhere in the genome. The gene trap transposon construct was transfected together with a transposase source (FP Tpase) into human HeLa cells. Some of the gene trap transposons insert into the introns and untranslated regions of genes, a fraction of which will be in the proper orientation so that a fusion transcript between the endogenous gene and the neo marker gene can be produced. Transfected cells were placed under zeocin and G418 selection. Several transposon insertions were cloned from antibiotic-resistant cell clones, and the insertions were mapped on human chromosomes using BLAST. All of these insertions occurred in genes. Integration of the gene trap vector into actively transcribed genes can act as a locus specific marker in living animals and will provide tags for identifying the disrupted genes. The constructs used and the results obtained in this experiment are shown in Figure 6.

### Example 7: Generation of transposases with increased transpositional activity

Introducing point mutation into the *Frog Prince* transposase gene with GENE MORPH (Stratagene) kit was based on a PCR mutagenesis approach. The conditions were set in a way that one point mutation is generated per gene in average. We were making a library of mutated transposase genes of about 10,000. The PCR products were cloned into a eukaryotic expression vector with a high efficiency (80-90 % of the clones contain a transposase gene). The plasmid DNA was purified from the clones with a pipetting robot (Tecan) in a 96 well format. The purified plasmid DNA was used to transfect human cultured cells in a high through put transposition assay. The mutant transposases were co-transfected with the transposon DNA containing a gene for neomycin resistence. The elevated number of the resistant colonies would indicate hyperactivity in the screen.
So far we have identified three clones that show hyperactivity. Clones AD8, BG8, and BG12 are estimated to have at least a three times higher activity as FP. We have all the reason to believe that the project would produce hyperactive transposases of 10-100- fold activity as compared to the wild-type protein.

### REFERENCES

Ivics, Z., Izsvák, Z., Minter, A. & Hackett, P. B. (1996). Identification of functional domains and evolution of Tc1-like transposable elements. Proc. Natl. Acad. Sci. U.S.A. 93, 5008-13.
Ivics, Z., Hackett, P.B., Plasterk, R.H. & Izsvak, Z. Molecular reconstruction of Sleeping Beauty, a Tcl-like transposon from fish, and its transposition in human cells. Cell 91, 501-510 (1997).
Izsvák, Zs., Ivics, Z. and Hackett, P.B. (1995). Characterization of a Tc1-like transposable element in zebrafish (Danio rerio). Mol. Gen. Genet. 247:312-322.
Izsvák, Z., Ivics, Z., and Plasterk, R. H. (2000) Sleeping Beauty, a wide host-range transposon vector for genetic transformation in vertebrates. J. Mol. Biol. 302, 93-102.
Lam, W. L., Seo, P., Robison, K., Virk, S. & Gilbert, W. (1996). Discovery of amphibian Tc1-like transposon families. J. Mol. Biol. 257, 359-66.
Luo, G., Ivics, Z., Izsvak, Z. & Bradley, A. (1998). Chromosomal transposition of a Tc1/mariner-like element in mouse embryonic stem cells. Proc Natl Acad Sci U S A 95, 10769-10773.
Miller, A.D. (1997). Development and applications of retroviral vectors. in Retroviruses (eds. Coffin, J.M., Hughes, S.H. & Varmus, H.E.) 843 pp. (Cold Spring Harbor Laboratory Press, New York,).
Plasterk, R. H., Izsvák, Z. & Ivics, Z. (1999). Resident aliens: the Tc1/mariner superfamily of transposable elements. Trend Genet. 15, 326-32.
Verma, I.M. and Somia, N. (1997). Gene therapy - promises, problems and prospects. Nature 389, 239-242.
Yant, S. R., Meuse, L., Chiu, W., Ivics, Z., Izsvak, Z., and Kay, M. A. (2000) Somatic integration and long-term transgene expression in normal and haemophilic mice using a DNA transposon system. Nat. Genet. 25, 35-41.

## Claims

1. A transposon-based DNA integration system comprising
(a) a transposon which is devoid of a polynucleotide encoding a functional transposase and which comprises a polynucleotide of interest, wherein the transposon comprises inverted repeats having a degree of identity with the repeats within SEQ ID NO: 2 and its inverted repeat, respectively, of at least 90%; and
(b) a transposase having at its N-terminus a DNA binding domain comprising the sequences of SEQ ID NO: 3 and 4 or having a degree of identity of at least 90 % to SEQ ID NO: 3 and/or 4; or
(c) a polynucleotide encoding the transposase of (b)

2. The transposon-based DNA integration system of claim 1 wherein the transposase comprises a bipartite nuclear localisation signal represented by SEQ ID NO: 5, and/or a catalytic domain with a DD(34)E signature represented by SEQ ID NO: 7 and/or a DNA-binding domain of SEQ ID NO: 6.

3. The transposon-based DNA integration system of claim 1 or 2 wherein said transposase has a degree of identity with the amino acid sequence of SEQ ID NO: 1 of at least 90 %.

4. The transposon-based DNA integration system of any one of claims 1 to 3 wherein the polynucleotide of interest is a gene.

5. The transposon-based DNA integration system of claim 4 wherein said gene is derived from a mammal, fish, amphibian, reptile or bird.

6. The transposon-based DNA integration system of claim 5 wherein said mammal is a human.

7. The transposon-based DNA integration system of any one of claims 1 to 6 wherein said polynucleotide of interest encodes a therapeutically active (poly)peptide.

8. The transposon-based DNA integration system of any one of claims 1 to 6 wherein said polynucleotide of interest transcribes into siRNA and encodes a selectable marker.

9. The transposon-based DNA integration system of any one of claims 1 to 6 wherein said polynucleotide of interest comprises a intron splice acceptor site and a selectable marker gene wherein said gene lacks the methionine start codon and contains a polyA addition signal.

10. The transposon-based DNA integration system of any one of claims 1 to 9 wherein said transposase has an enhanced transposase activity.

11. The transposon-based DNA integration system of any one of claims 1 to 10 wherein the transposon of (a) and/or the polynucleotide of (c) is comprised in at least one vector.

12. The transposon-based DNA integration system of claim 11 wherein said vector is a plasmid.

13. A host cell transfected or transformed with the transposon-based DNA integration system of claim 11 or 12.

14. A non-human transgenic animal comprising the transposon of (a) or the transposon-based DNA integration system of claim 11 or 12 stably integrated into its genome.

15. Use of the transposon-based DNA integration system of any of claims 1 to 12 for the preparation of a medicament, whereby a polynucleotide of interest is transferred into cells of a vertebrate by introducing the transposon-based DNA integration system into said cells.

16. Use of claim 15 wherein the transfer is effected in vitro.

17. Use of claim 15 wherein the transfer is effected in vivo.

18. Use of claim 16 further whereby
(a) cells are selected wherein said polynucleotide is stably integrated into the chromosomes of said cells; and whereby
(b) said cells or cells derived from said cells are introduced or reintroduced into a vertebrate of the same species.

19. Use of claim 16 or 18 wherein the vertebrate into which said cells are reintroduced is the vertebrate from which the cells were taken prior to said transfer.

20. Use of any one of claims 15 to 19 wherein said transfer is effected by means of a gene gun, or by means of a gene transfer mediated or assisted by liposomes, polyethyleneimine, adenovirus-polylysine-DNA complexes, lipofection, electroporation, transfection/transduction or infection.

21. Use of claim 20 wherein said infection or transduction is mediated or assisted by recombinant retrovirus, recombinant adenovirus, recombinant herpes virus or recombinant adeno-associated virus.

22. Use of any one of claims 15 to 21 wherein said cells are somatic cells.

23. Use according to claim 22 wherein the vertebrate is a mammal, fish, amphibian, reptile or bird.

24. Use of claim 23 wherein said mammal is a human.

25. Use of any one of claims 15 to 21 wherein said cells are non-human germ line cells.

26. Use according to claim 25 wherein the vertebrate is a non-human mammal, fish, amphibian, reptile or bird.

27. Use of claim 25 or 26, whereby after reintroducing said non-human germ line cells or a cell derived from said germ line cell into a vertebrate of the same species, a transgenic vertebrate is grown wherein said vertebrate or transgenic vertebrate is not a human.

28. Use of any one of claims 15, 17 and 25 to 27 wherein said introduction or reintroduction is mediated or effected by sperm, microinjection or by means of a gene gun.

29. An in-vitro method of effecting RNAi comprising
(a) stably introducing the transposon being part of the transposon-based DNA integration system of claim 8 into a cell;
(b) selecting for cells expressing the selectable marker; and
(c) assessing whether the transcription/translation of the desired gene is effected by RNAi.

30. An in-vitro method of gene trapping genes comprising
(a) introducing the transposon-based DNA integration system of claim 9 into a cell; and
(b) assessing for the expression of a selectable marker wherein expression of a selectable marker is indicative of integration of the transposon into a transcibed gene of the cell.

31. The in vitro method of claim 30 further comprising
(c) identifying the disrupted gene by means of the integrated transposon as a tag.

32. A transposon **characterized in that** it comprises or consists of inverted repeats having a degree of identity with the repeats within SEQ ID NO: 2 and its inverted repeat, respectively, of at least 90%; and a transposase having at its N-terminus a DNA binding domain comprising the sequences of SEQ ID NO: 3 and 4.

33. A composition comprising the transposon-based DNA integration system of any one of claims 1 to 12.

34. The composition of claim 33 which is a pharmaceutical composition.

35. The composition of claim 33 which is a diagnostic composition or a kit.

## Patentansprüche

1. DNA-Integrationssystem auf Transposon-Basis, umfassend
(a) ein Transposon, welchem ein eine funktionelle Transposase kodierendes Polynukleotid fehlt und welches ein interessierendes Polynukleotid umfasst, wobei das Transposon invertierte Wiederholungen ("Repeats") enthält, welche mit den Wiederholungen innerhalb der SEQ ID NO: 2 bzw. deren invertierter Wiederholung einen Identitätsgrad von wenigstens 90 % aufweisen; und
(b) eine Transposase, welche an ihrem N-Terminus eine DNA-Bindungsdomäne aufweist, welche die Sequenzen von SEQ ID NO: 3 und 4 umfasst oder zu SEQ ID NO:3 und/oder 4 einen Identitätsgrad von wenigstens 90 % aufweist; oder
(c) ein Polynukleotid, welches die Transposase von (b) kodiert.

2. DNA-Integrationssystem auf Transposon-Basis nach Anspruch 1, wobei die Transposase ein zweigeteiltes (bipartites) Kernlokalisationssignal gemäß SEQ ID NO:5 und/oder eine katalytische Domäne mit einer DD(34)E-Signatur gemäß SEQ ID NO: 7 und/oder eine DNA-Bindungsdomäne gemäß SEQ ID NO: 6 umfasst.

3. DNA-Integrationssystem auf Transposon-Basis nach Anspruch 1 oder 2, wobei die Transposase mit der Aminosäuresequenz von SEQ ID NO:1 einen Identitätsgrad von wenigstens 90 % aufweist.

4. DNA-Integrationssystem auf Transposon-Basis nach irgendeinem der Ansprüche 1 bis 3, wobei das interessierende Polynukleotid ein Gen ist.

5. DNA-Integrationssystem auf Transposon-Basis nach Anspruch 4, wobei das Gen von einem Säugetier, einem Fisch, einer Amphibie, einem Reptil oder einem Vogel stammt.

6. DNA-Integrationssystem auf Transposon-Basis nach Anspruch 5, wobei das Säugetier ein Mensch ist.

7. DNA-Integrationssystem auf Transposon-Basis nach irgendeinem der Ansprüche 1 bis 6, wobei das interessierende Polynukleotid ein therapeutisch wirksames (Poly-) Peptid kodiert.

8. DNA-Integrationssystem auf Transposon-Basis nach irgendeinem der Ansprüche 1 bis 6, wobei das interessierende Polynukleotid zu siRNA transkribiert wird und einen selektierbaren Marker kodiert.

9. DNA-Integrationssystem auf Transposon-Basis nach irgendeinem der Ansprüche 1 bis 6, wobei das interessierende Polynukleotid eine Intron-Splice-Akzeptor-Stelle und ein selektierbares Markergen umfasst, wobei diesem Gen das Methionin-Startcodon fehlt und es ein PolyA-Additionssignal enthält.

10. DNA-Integrationssystem auf Transposon-Basis nach irgendeinem der Ansprüche 1 bis 9, wobei die Transposase eine verstärkte Transposase-Aktivität aufweist.

11. DNA-Integrationssystem auf Transposon-Basis nach irgendeinem der Ansprüche 1 bis 10, wobei das Transposon von (a) und/oder das Polynukleotid von (c) in wenigstens einem Vektor enthalten ist/sind.

12. DNA-Integrationssystem auf Transposon-Basis nach Anspruch 11, wobei der Vektor ein Plasmid ist.

13. Wirtszelle, welche mit dem DNA-Integrationssystem auf Transposon-Basis von Anspruch 11 oder 12 transfiziert oder transformiert ist.

14. Nicht-humanes transgenes Tier, welches das Transposon von (a) oder das DNA-Integrationssystem auf Transposon-Basis nach Anspruch 11 oder 12 stabil in sein Genom integriert enthält.

15. Verwendung des DNA-Integrationssystems auf Transposon-Basis nach irgendeinem der Ansprüche 1 bis 12 zur Herstellung eines Arzneimittels, wobei ein interessierendes Polynukleotid in die Zellen eines Vertebraten transferiert wird, indem das DNA-Integrationssystem auf Transposon-Basis in die Zellen eingeführt wird.

16. Verwendung nach Anspruch 15, wobei der Transfer *in vitro* erfolgt.

17. Verwendung nach Anspruch 15, wobei der Transfer *in vivo* erfolgt.

18. Verwendung nach Anspruch 16, wobei außerdem
(a) Zellen ausgewählt werden, wobei das Polynucleotid stabil in die Chromosomen dieser Zellen integriert ist, und wobei
(b) diese Zellen oder von diesen Zellen abstammende Zellen in einen Vertebraten der gleichen Spezies eingeführt oder wieder eingeführt werden.

19. Verwendung nach Anspruch 16 oder 18, wobei der Vertebrat, in den die Zellen wieder eingeführt werden, der Vertebrat ist, von dem die Zellen vor dem Transfer entnommen wurden.

20. Verwendung nach irgendeinem der Ansprüche 15 bis 19, wobei der Transfer mittels einer Genpistole ("Gene-Gun") oder mittels eines durch Liposomen, Polyethylenimin, Adenovirus-Polylysin-DNA-Komplexe, Lipofektion, Elektroporation, Transfektion/Transduktion oder Infektion vermittelten oder unterstützten Gentransfers bewirkt wird.

21. Verwendung gemäß Anspruch 20, wobei die Infektion oder Transduktion durch rekombinantes Retrovirus, rekombinantes Adenovirus, rekombinantes Herpesvirus oder rekombinantes mit Adenovirus verwandtes Virus ("adeno-associated virus") vermittelt oder unterstützt wird.

22. Verwendung nach irgendeinem der Ansprüche 15 bis 21, wobei die Zellen somatische Zellen sind.

23. Verwendung gemäß Anspruch 22, wobei der Vertebrat ein Säugetier, ein Fisch, eine Amphibie, ein Reptil oder ein Vogel ist.

24. Verwendung gemäß Anspruch 23, wobei das Säugetier ein Mensch ist.

25. Verwendung nach irgendeinem der Ansprüche 15 bis 21, wobei die Zellen nicht-humane Keimbahnzellen sind.

26. Verwendung gemäß Anspruch 25, wobei der Vertebrat ein nicht-humanes Säugetier, ein Fisch, eine Amphibie, ein Reptil oder ein Vogel ist.

27. Verwendung nach Anspruch 25 oder 26, wobei nach Wiedereinführung der nicht-humanen Keimbahnzellen oder von Zellen, die von den Keimbahnzellen abstammen, in einen Vertebraten der gleichen Spezies ein transgener Vertebrat herangezogen wird, wobei dieser Vertebrat oder transgene Vertebrat kein Mensch ist.

28. Verwendung nach irgendeinem der Ansprüche 15, 17 und 25 bis 27, wobei die Einführung oder Wiedereinführung durch Sperma, Mikroinjektion oder mittels einer Genpistole vermittelt oder bewirkt wird.

29. In-Vitro-Verfahren zum Bewirken von RNAi, umfassend
(a) das stabile Einführen des Transposons, welches einen Teil des DNA-Integrationssystems auf Transposon-Basis nach Anspruch 8 darstellt, in eine Zelle;
(b) das Selektieren nach Zellen, welche den selektierbaren Marker exprimieren; und
(c) das Feststellen, ob die Transkription/Translation des gewünschten Gens durch RNAi beeinflusst wird.

30. In-Vitro-Verfahren zum Gen-Trapping von Genen, umfassend
(a) das Einführen des DNA-Integrationssystems auf Transposon-Basis nach Anspruch 9 in eine Zelle; und
(b) das Feststellen der Expression eines selektierbaren Markers, wobei die Expression eines selektierbaren Markers indikativ für die Integration des Transposons in ein transkribiertes Gen der Zelle ist.

31. In-Vitro-Verfahren nach Anspruch 30, weiterhin umfassend
(c) die Identifizierung des unterbrochenen Gens mittels des integrierten Transposons als "Tag".

32. Transposon, **dadurch gekennzeichnet, dass** es aus invertierten Wiederholungen ("Repeats"), welche mit den Wiederholungen innerhalb von SEQ ID NO:2 bzw. mit deren invertierter Wiederholung einen Identitätsgrad von wenigstens 90% aufweisen, und einer Transposase, welche an ihrem N-Terminus eine DNA-Bindungsdomäne aufweist, welche die Sequenzen von SEQ ID NO: 3 und 4 umfasst, besteht oder diese enthält.

33. Zusammensetzung, welche das DNA-Integrationssystem auf Transposon-Basis nach irgendeinem der Ansprüche 1 bis 12 umfasst.

34. Zusammensetzung nach Anspruch 33, welche eine pharmazeutische Zusammensetzung darstellt.

35. Zusammensetzung nach Anspruch 33, welche eine diagnostische Zusammensetzung oder einen Kit darstellt.

## Revendications

1. Système d'intégration d'ADN à base de transposons comportant
(a) un transposon qui est dépourvu d'un polynucléotide codant pour une transposase fonctionnelle et qui comporte un polynucléotide d'intérêt, le transposon comportant des répétitions inversées ayant un degré d'identité avec les répétitions dans la SEQ ID N° : 2 et sa répétition inversée, respectivement, d'au moins 90 %, et
(b) une transposase possédant au niveau de son N-terminal, un domaine de liaison de l'ADN comportant les séquences de SEQ ID N° : 3 et 4 ou ayant un degré d'identité d'au moins 90 % à la SEQ ID N° : 3 et/ou 4, ou
(c) un polynucléotide codant pour la transposase de (b).

2. Système d'intégration d'ADN à base de transposons selon la revendication 1, dans lequel la transposase comporte un signal de localisation nucléaire bipartite représenté par la SEQ ID N° : 5, et/ou un domaine catalytique avec une signature DD(34)E représentée par la SEQ ID N° : 7 et/ou un domaine de liaison de l'ADN de la SEQ ID N° : 6.

3. Système d'intégration d'ADN à base de transposons selon la revendication 1 ou 2, dans lequel ladite transposase a un degré d'identité avec la séquence d'acides aminés de la SEQ ID N° : 1 d'au moins 90 %.

4. Système d'intégration d'ADN à base de transposons selon l'une quelconque des revendications 1 à 3, dans lequel le polynucléotide d'intérêt est un gène.

5. Système d'intégration d'ADN à base de transposons selon la revendication 4, dans lequel ledit gène est dérivé d'un mammifère, d'un poisson, d'un amphibien, d'un reptile ou d'un oiseau.

6. Système d'intégration d'ADN à base de transposons selon la revendication 5, dans lequel ledit mammifère est un être humain.

7. Système d'intégration d'ADN à base de transposons selon l'une quelconque des revendications 1 à 6, dans lequel ledit polynucléotide d'intérêt code pour un (poly)peptide thérapeutiquement actif.

8. Système d'intégration d'ADN à base de transposons selon l'une quelconque des revendications 1 à 6, dans lequel ledit polynucléotide d'intérêt transcrit en siARN et code pour un marqueur sélectionnable.

9. Système d'intégration d'ADN à base de transposons selon l'une quelconque des revendications 1 à 6, dans lequel ledit polynucléotide d'intérêt comporte un site accepteur d'épissage de l'intron et un gène marqueur sélectionnable dans lequel il manque audit gène le codon méthionine initiateur et ledit gène contient un signal d'addition de polyA.

10. Système d'intégration d'ADN à base de transposons selon l'une quelconque des revendications 1 à 9, dans lequel ladite transposase a une activité transposase améliorée.

11. Système d'intégration d'ADN à base de transposons selon l'une quelconque des revendications 1 à 10, dans lequel le transposon de (a) et/ou le polynucléotide de c) est inclus dans au moins un vecteur.

12. Système d'intégration d'ADN à base de transposons selon la revendication 11, dans lequel ledit vecteur est un plasmide.

13. Cellule hôte transfectée ou transformée avec le système d'intégration d'ADN à base de transposons selon la revendication 11 ou 12.

14. Animal transgénique non humain comportant le transposon de (a) ou le système d'intégration d'ADN à base de transposons de la revendication 11 ou 12 intégré de manière stable dans son génome.

15. Utilisation du système d'intégration d'ADN à base de transposons selon l'une quelconque des revendications 1 à 12 pour la préparation d'un médicament, de sorte qu'un polynucléotide d'intérêt est transféré dans des cellules d'un vertébré en introduisant le système d'intégration d'ADN à base de transposons dans lesdites cellules.

16. Utilisation selon la revendication 15, dans laquelle le transfert est réalisé in vitro.

17. Utilisation selon la revendication 15, dans lequel le transfert est réalisé in vivo.

18. Utilisation selon la revendication 16, de sorte qu'en outre
(a) on sélectionne des cellules dans lesquelles ledit polynucléotide est intégré de manière stable dans les chromosomes desdites cellules, et de sorte que
(b) lesdites cellules ou des cellules dérivées desdites cellules sont introduites ou réintroduites dans un vertébré de la même espèce.

19. Utilisation selon la revendication 16 ou 18, dans laquelle le vertébré dans lequel lesdites cellules sont réintroduites est le vertébré sur lequel les cellules ont été prélevées avant ledit transfert.

20. Utilisation selon l'une quelconque des revendications 15 à 19, dans laquelle ledit transfert est réalisé au moyen d'un canon à gènes, ou au moyen d'un transfert de gènes médié ou assisté par liposomes, polyéthylèneimine, complexes adénovirus-polylysine-ADN, lipofection, électroporation, transfection/transduction ou infection.

21. Utilisation selon la revendication 20, dans laquelle ladite infection ou transduction est médiée ou assistée par rétrovirus recombinant, adénovirus recombinant, herpès virus recombinant ou virus adéno-associé recombinant.

22. Utilisation selon l'une quelconque des revendications 15 à 21, dans laquelle lesdites cellules sont des cellules somatiques.

23. Utilisation selon la revendication 22, dans laquelle le vertébré est un mammifère, un poisson, un amphibien, un reptile ou un oiseau.

24. Utilisation selon la revendication 23, dans laquelle ledit mammifère est un être humain.

25. Utilisation selon l'une quelconque des revendications 15 à 21, dans laquelle lesdites cellules sont des cellules de lignée germinale non humaines.

26. Utilisation selon la revendication 25, dans laquelle le vertébré est un mammifère non humain, un poisson, un amphibien, un reptile ou un oiseau.

27. Utilisation selon la revendication 25 ou 26, de sorte qu'après réintroduction desdites cellules de lignée germinale non humaines ou d'une cellule dérivée de ladite cellule de lignée germinale dans un vertébré de la même espèce, un vertébré transgénique se développe, ledit vertébré ou vertébré transgénique n'étant pas un être humain.

28. Utilisation selon l'une quelconque des revendications 15, 17 et 25 à 27, dans laquelle ladite introduction ou réintroduction est médiée ou réalisée par du sperme, une microinjection ou au moyen d'un canon à gènes.

29. Procédé in vitro pour réaliser une ARNi comportant les étapes consistant à :
(a) introduire de manière stable le transposon faisant partie du système d'intégration d'ADN à base de transposons de la revendication 8 dans une cellule,
(b) sélectionner des cellules exprimant le marqueur sélectionnable, et
(c) évaluer si oui ou non la transcription/traduction du gène voulu est réalisée par l'ARNi.

30. Procédé in vitro de piégeage de gènes comportant les étapes consistant à :
(a) introduire le système d'intégration d'ADN à base de transposons de la revendication 9 dans une cellule, et
(b) évaluer l'expression d'un marqueur sélectionnable, l'expression d'un marqueur sélectionnable étant indicative de l'intégration du transposon dans un gène transcrit de la cellule.

31. Procédé in vitro selon la revendication 30 comportant également l'étape consistant à :
(c) identifier le gène interrompu au moyen du transposon intégré utilisé en tant qu'étiquette.

32. Transposon **caractérisé en ce qu'**il comporte ou est constitué de répétitions inversées ayant un degré d'identité avec les répétitions dans la SEQ ID N° : 2 et sa répétition inversée, respectivement, d'au moins 90 %, et une transposase possédant au niveau de son N-terminal, un domaine de liaison de l'ADN comportant les séquences de SEQ ID N° : 3 et 4.

33. Composition comportant le système d'intégration d'ADN à base de transposons de l'une quelconque des revendications 1 à 12.

34. Composition selon la revendication 33 qui est une composition pharmaceutique.

35. Composition selon la revendication 33 qui est une composition de diagnostic ou un kit.


### SEQUENCE LISTING

<110> Max-Delbrück-Centrum für Molekulare Medizin Netherland Cancer Institute
<120> The Frog Prince, a Transposon Vector for Gene Transfer in Vertebrates
<130> H1869 PCT
<150> 102 24 242.9
   <151> 2002-05-29
<160> 7
<170> PatentIn version 3.1
<210> 1
   <211> 340
   <212> PRT
   <213> Rana pipiens
<400> 1
   Met Pro Arg Pro Lys Glu Ile Gln Glu Gln Leu Arg Lys Lys Val Ile
   1               5                   10                  15
   Glu Ile Tyr Gln Ser Gly Lys Gly Tyr Lys Ala Ile Ser Lys Ala Leu
               20                  25                  30
   Gly Ile Gln Arg Thr Thr Val Arg Ala Ile Ile His Lys Trp Arg Arg
           35                  40                  45
   His Gly Thr Val Val Asn Leu Pro Arg Ser Gly Arg Pro Pro Lys Ile
       50                  55                  60
   Thr Pro Arg Ala Gln Arg Arg Leu Ile Gln Glu Val Thr Lys Asp Pro
   65                  70                  75                  80
   Thr Thr Thr Ser Lys Glu Leu Gln Ala Ser Leu Ala Ser Val Lys Val
                   85                  90                  95
   Ser Val His Ala Ser Thr Ile Arg Lys Arg Leu Gly Lys Asn Gly Leu
               100                 105                 110
   His Gly Arg Val Pro Arg Arg Lys Pro Leu Leu Ser Lys Lys Asn Ile
           115                 120                 125
   Lys Ala Arg Leu Asn Phe Ser Thr Thr His Leu Asp Asp Pro Gln Asp
       130                 135                 140
   Phe Trp Asp Asn Ile Leu Trp Thr Asp Glu Thr Lys Val Glu Leu Phe
   145                 150                 155                 160
   Gly Arg Cys Val Ser Lys Tyr Ile Trp Arg Arg Arg Asn Thr Ala Phe
                   165                 170                 175
   His Lys Lys Asn Ile Ile Pro Thr Val Lys Tyr Gly Gly Gly Ser Val
               180                 185                 190
   Met Val Trp Gly Cys Phe Ala Ala Ser Gly Pro Gly Arg Leu Ala Val
           195                 200                 205
   Ile Lys Gly Thr Met Asn Ser Ala Val Tyr Gln Glu Ile Leu Lys Glu
       210                 215                 220
   Asn Val Arg Pro Ser Val Arg Val Leu Lys Leu Lys Arg Thr Trp Val
   225                 230                 235                 240
   Leu Gln Gln Asp Asn Asp Pro Lys His Thr Ser Lys Ser Thr Thr Glu
                   245                 250                 255
   Trp Leu Lys Lys Asn Lys Met Lys Thr Leu Glu Trp Pro Ser Gln Ser
               260                 265                 270
   Pro Asp Leu Asn Pro Ile Glu Met Leu Trp Tyr Asp Leu Lys Lys Ala
           275                 280                 285
   Val His Ala Arg Lys Pro Ser Asn Val Thr Glu Leu Gly Gln Phe Cys
       290                 295                 300
   Lys Asp Glu Trp Ala Lys Ile Pro Pro Gly Arg Cys Lys Ser Leu Ile
   305                 310                 315                 320
   Ala Arg Tyr Arg Lys Arg Leu Val Ala Val Val Ala Ala Lys Gly Gly
                   325                 330                 335
   Pro Thr Ser Tyr
               340
<210> 2
   <211> 250
   <212> DNA
   <213> Rana pipiens
<400> 2
   cagtggtgtg aaaaagtgtt tgcccccttc ctcatttcct gttcctttgc atgtttgtca      60
   cacttaagtg tttcggaaca tcaaaccaat ttaaacaata gtcaaggaca acacaagtaa     120
   acacaaaatg caatttgtaa atgaaggtgt ttattattaa aggtgaaaaa aaatccaaac     180
   catcatggcc ctgtgtgaaa aagtgattgc cccccttgtt aaaacatact ataactgtgg      240
   ttgtccacac                                                            250
<210> 3
   <211> 40
   <212> PRT
   <213> Rana pipiens
<400> 3
   Lys Glu Ile Gln Glu Gln Leu Arg Lys Lys Val Ile Glu Ile Tyr Gln
   1               5                   10                  15
   Ser Gly Lys Gly Tyr Lys Ala Ile Ser Lys Ala Leu Gly Ile Gln Arg
               20                  25                  30
   Thr Thr Val Arg Ala Ile Ile His
           35                  40
<210> 4
   <211> 42
   <212> PRT
   <213> Rana pipiens
<400> 4
   Gln Arg Arg Leu Ile Gln Glu Val Thr Lys Asp Pro Thr Thr Thr Ser
   1 5 10 15
   Lys Glu Leu Gln Ala Ser Leu Ala Ser Val Lys Val Ser Val His Ala
   20 25 30
   Ser Thr Ile Arg Lys Arg Leu Gly Lys Asn
   35 40
<210> 5
   <211> 18
   <212> PRT
   <213> Rana pipiens
<400> 5
   Arg Lys Arg Leu Gly Lys Asn Gly Leu His Gly Arg Val Pro Arg Arg
   1 5 10 15
   Lys Pro
<210> 6
   <211> 123
   <212> PRT
   <213> Rana pipiens
<400> 6
   Met Pro Arg Pro Lys Glu Ile Gln Glu Gln Leu Arg Lys Lys Val Ile
   1 5 10 15
   Glu Ile Tyr Gln Ser Gly Lys Gly Tyr Lys Ala Ile Ser Lys Ala Leu
   20 25 30
   Gly Ile Gln Arg Thr Thr Val Arg Ala Ile Ile His Lys Trp Arg Arg
   35 40 45
   His Gly Thr Val Val Asn Leu Pro Arg Ser Gly Arg Pro Pro Lys Ile
   50 55 60
   Thr Pro Arg Ala Gln Arg Arg Leu Ile Gln Glu Val Thr Lys Asp Pro
   65 70 75 80
   Thr Thr Thr Ser Lys Glu Leu Gln Ala Ser Leu Ala Ser Val Lys Val
   85 90 95
   Ser Val His Ala Ser Thr Ile Arg Lys Arg Leu Gly Lys Asn Gly Leu
   100 105 110
   His Gly Arg Val Pro Arg Arg Lys Pro Leu Leu
   115 120
<210> 7
   <211> 127
   <212> PRT
   <213> Rana pipiens
<400> 7
   Asp Glu Thr Lys Val Glu Leu Phe Gly Arg Cys Val Ser Lys Tyr Ile
   1 5 10 15
   Trp Arg Arg Arg Asn Thr Ala Phe His Lys Lys Asn Ile Ile Pro Thr
   20 25 30
   Val Lys Tyr Gly Gly Gly Ser Val Met Val Trp Gly Cys Phe Ala Ala
   35 40 45
   Ser Gly Pro Gly Arg Leu Ala Val Ile Lys Gly Thr Met Asn Ser Ala
   50 55 60
   Val Tyr Gln Glu Ile Leu Lys Glu Asn Val Arg Pro Ser Val Arg Val
   65 70 75 80
   Leu Lys Leu Lys Arg Thr Trp Val Leu Gln Gln Asp Asn Asp Pro Lys
   85 90 95
   His Thr Ser Lys Ser Thr Thr Glu Trp Leu Lys Lys Asn Lys Met Lys
   100 105 110
   Thr Leu Glu Trp Pro Ser Gln Ser Pro Asp Leu Asn Pro Ile Glu
           115                 120                 125